# EUROPEAN PATENT APPLICATION

(11) **EP 4 360 599 A1**
(43) Date of publication of application: **01.05.2024**
(21) Application number: 22306651.5
(22) Date of filing: 31.10.2022
(51) Int. Cl.: A61F 5/44, A61F 5/445

(54) **PROTECTING AND CONNECTING DEVICE OF AT LEAST ONE STOMA SITE EMERGING OUT OF A PATIENT'S SKIN**

(71) Applicant: Centre Hospitalier Universitaire De Toulouse, 31059 Toulouse Cedex 9 (FR); Leartiker Institute, 48270 Markina-Xemein, Biscay (ES)
(72) Inventor: MAS, Emmanuel, 31700 Beauzelle (FR); ABBO, Olivier, 31300 Toulouse (FR); MARBACH, Clothilde, 31000 Toulouse (FR); ZALDUA HUICI, Ane Miren, 20006 Donostia, Gipuzkoa (ES); GEREKA GOIENETXE, Ainitze, 20808 Getaria, Gipuzkoa (ES); LARREATEGI MAKATZAGA, Pablo, 20600 Eibar, Gipuzkoa (ES); URTAZA SARRIONANDIA, Uzuri, 48220 Abadiño, Biscay (ES)
(74) Representative: BARRE LAFORGUE

(57) **Abstract**

The invention relates to a protecting and connecting device (10) of at least one stoma site emerging out of a patient's skin, the protecting and connecting device comprising:
- a shield (30) that comprises a wall (31) delimited by a peripheral outline (32), the shield generating an inside compartment protecting the site of said stoma,
- an outskirt (40) surrounding at least partially peripheral outline of the shield, wherein a lower face of the outskirt comprises an adhesive layer for sealing the shield onto the patient's skin, and
- at least one receiving hole (50, 51) through the wall of the shield, said receiving hole defining a passage for at least one probe (60, 61).

According to the invention, the protecting and connecting device comprises a tab (70) providing a grasp mean for unsealing the shield from the patient's skin.

## Description

### [FIELD OF THE INVENTION]

The invention relates to the field of medical devices and more particularly of devices used in surgical activities. Indeed, the invention encompasses the protection of at least one stoma emerging out of a patient's skin. In particular, the invention can be related to a protecting and connecting device for the treatment of the intestinal atresia. Therefore, the invention may concern a protecting and connecting device of a stoma site emerging out from the patient's skin.

Intestinal atresia is a congenital malformation that causes bowel obstruction of the newborn and threatens their survival. Intestinal atresia is characterized by an enlarged bowel upstream the atresia and a smaller bowel downstream. Whenever this malformation is diagnosed, a surgery is required to treat the intestine of the newborn. This kind of surgery is performed at birth and involves shunting, that-is-to say making a derivation of the intestine outside of the newborn's body while the intestine is treated by the surgeons. Generally, the derivation is kept in place for more than three months in order to permit the healing of the intestine after the surgery. More precisely, the small intestine is derived through two stomas emerging from the newborn's skin. Intestinal secretions are collected within a stoma bag. After 3 months, a second surgery is performed to restore intestine continuity.

For now, the intestinal secretions directly go from the stoma to the bag and a tube is setup once or twice a date after several days to fill the downstream part of the intestine

The treatment of the intestinal atresia is relatively cumbersome and delicate. More specifically, it involves healing the atresia by suturing the upstream and downstream intestinal segments to restore continuity of the intestine. To do this, the surgeons need to protect the suturing site by creating a temporary derivation of the intestinal secretions.

Such a surgical procedure implies one first surgery to make the derivation of the intestine and the instatement of a parenteral nutrition through a central line, then a healing period which lasts several months during which the derivation and the parenteral nutrition remain in place, and finally a second surgery for restoring the continuity of the intestine. One of the main issues pertains to protect the skin around the stomas from intestinal secretions but also to achieve a clean derivation of the intestine in order to maintain a regular flow of the intestinal secretions through the derivation throughout the initial surgery and the healing period.

### [BACKGROUND OF THE INVENTION]

There does not exist any device specifically designed for the treatment of the intestinal atresia. Most of the time, the surgeons adapt devices that have been designed for other applications. In this case, the surgeons usually make an adapted version of an ostomy bag for carrying out the intestinal atresia surgery.

However, even the dimensions of the ostomy bags made for children are too large for providing an adapted solution to be connected onto stomas setup onto newborns, and therefore the surgeons must adapt the size of the ostomy bag which is not designed for a newborn. Moreover, the ostomy bag comprises only one opening to be connected to a stoma. Unfortunately, the first stoma and the second stoma cannot be independently connected to the ostomy bag, in consequence, it is directly connected to both stomas through the same opening. For this reason, the bag is filled by intestinal secretions that flow from a drain probe. For feeding the downstream stoma, a tube is installed between the ostomy bag and the downstream stoma once or twice a date after several days.

Such a setup presents many drawbacks. For example, the bag containing intestinal secretions does remain in contact, through the wall of the bag, with the newborn skin for a very long time, and thus causes some damages or irritations to the skin. Another issue is related to the ostomy bag that establishes a retention tank of the intestinal secretions, which does not enable to provide a constant flow between the drain probe and the feeding probe of the foregoing setup. In addition, given that both stomas are connected to the ostomy bag by the same opening, the sealing of the derivation is not reliable and cannot avoid leaks of the secretions, which also involves a hygienic issue regarding both stomas. Indeed, because of leakages, the "one-peace" stoma bag must be replaced several times a day.

Some solutions to protect a stoma have been described in other medical applications that notably imply a drain probe. For example, document EP 1 140 271 discloses a medical device comprising a collecting chamber that is sealed onto the patient's skin. In practice, the medical device includes an outskirt that includes a lower face coated by an adhesive layer which allows to seal the medical device onto the patient's skin. The collecting chamber comprises an inclined top face on which two receiving orifices are arranged. A first orifice is used as a guide to insert a drain through the patient's body, and the second orifice, which is larger than the first orifice is configured to be connected to a collecting bag for accumulating the secretion fluids.

Such a medical device is not convenient for protecting the skin around one stoma but rather adapted to collect the body secretions into the collecting chamber. It does not permit to make a clean derivation of the intestine outside of the patient's body nor to replace the device without disconnecting the derivation. Moreover, such a device cannot be easily removed several times per day in order to clean up both stomas while the derivation remains in place outside the patient's body.

Document US 2017/021134 describes a specific device to setup an umbilical central catheter onto a newborn. To do this, a rigid dome is used as a shield to protect the emerging site of the catheter. At its base, the rigid dome is surrounded by a thin annular portion that comprises an adhesive layer intended to contact with the patient's skin. To protect the skin of the newborn from injury, the annular portion is more flexible than the rigid dome. In addition, a pathway for the catheter is located of the top of the dome and this pathway can comprise two channels.

Such a device cannot be transposed to make a derivation of the intestine for the purpose of intestinal atresia surgery mainly because the protecting device must provide a derivation of the intestine transit outside of the body for several months. In addition, this device cannot be frequently and easily removed for cleaning up the stoma while the derivation of the intestine transit is kept in place outside of the body.

The invention aims to provide a solution to address the drawbacks of the prior art.

### [SUMMARY OF THE INVENTION]

In particular, the invention aims to provide a solution for establishing and maintaining sterile conditions around one or two stomas emerging out from the patient's skin but also for avoiding fluid leakages and enabling to frequently clean up the stomas while the derivation remains in place outside of the body.

The invention thus relates to a protecting and connecting device of at least one stoma site emerging out of a patient's skin, the protecting and connecting device comprising:
- a shield that comprises a wall delimited by a peripheral outline, the shield generating an inside compartment protecting the site of said stoma,
- an outskirt surrounding at least partially peripheral outline of the shield, wherein a lower face of the outskirt comprises an adhesive layer for sealing the shield onto the patient's skin,
- at least one receiving hole through the wall of the shield, said receiving hole defining a passage for at least one probe,
wherein the protecting and connecting device comprises a tab providing a grasp mean for unsealing the shield from the patient's skin.

The shield and the outskirt provide a hermetic compartment sealed to the patient's skin for protecting one or both stoma sites while the hole generates a passage for setup at least one probe connected to one tissue inside of the patient. The hermetic compartment allows to protect both stomas for several days and thus and thus reduce the frequency of cleaning process. The probe also protects the skin by allowing to drain secretions outside of the body, but the probe can also be used for enteral feeding of the patient. In the meantime, the tab provides a grasp mean that allows to softly unseal the outskirt for example to clean up the stoma while the probe is remaining connected to one tissue like the intestine, which thus preserves the newborn's skin from being injured by the operation of unsealing the outskirt which must be repeated several times all over the healing period. Therefore, the invention provides a solution suitable for treating the newborns diagnosed with intestine atresia.

The invention further allows to improve the comfort of the newborn during the surgery and the recovery period, and to reduce the time of the recovery period.

According to one embodiment, the receiving hole can further have a reinforcing structure and a notch arranged through a peripheral edge of said receiving hole, the notch being mobile between a closed position and an opened position. Such notch mounted on the reinforcing structure thus permits to engage and disengage the probe in the receiving hole without disconnecting the probe from a separate medical equipment to which the protecting and connecting device is operatively coupled.

According to another embodiment, the reinforcing structure includes a cylinder which extends from the peripheral edge of the receiving hole toward the exterior of the shield. This provides the advantage that enables to hold the probe through the wall of the shield.

According to a particular embodiment, the cylinder can include ridges located on an inside wall of the cylinder. The ridges thus contribute to adjust the tube of the probe with the receiving hole in order to reduce the slipping between the receiving hole and the probe.

According to one embodiment, the tab can be connected to one radial portion of the peripheral edge of said receiving hole so that the tab actuates the notch between the closed position and the opened position. In application where the outside connection of the probe is kept in place, an engagement and a disengagement with the protecting and connecting device are facilitated according to this embodiment. Indeed, the actuation of the tab allows to unseal but also to engage or disengage the probe into or from, respectively, the receiving hole.

According to one embodiment, the outskirt comprising a lateral opening and the tab is configured to face and overlap at least the lateral opening for establishing the sealing of the inside compartment. The lateral opening allows to softly unseal the outskirt when the tab is lifted. Firstly, the tab is unsealed from the patient's skin, then it causes the progressive unsealing of the outskirt. According to another embodiment, the lateral opening is in communication with said receiving hole and more particularly, the lateral opening connects said receiving hole through the notch of the reinforced structure. Thereby, the probe can be engaged and disengaged by the lateral opening and the notch without removing the connection of the probe outside the patient's body.

According to one embodiment, the tab includes a lower face that is coated by an adhesive layer, which enables to seal the shield to the patient's skin. Preferably, the adhesive layer can comprise a skin careful adhesive, that-is-to-say an adhesive compatible with the thinness and fragility of a newborn' skin. This contributes to preserve the skin integrity of the patient. In addition, in one embodiment the adhesive layer can be protected by a removal liner. The removal liner protects the adhesive layer during the transport and the storage of the protecting and connecting device.

According to one embodiment, the tab can further include:
- a central portion that overlaps and faces the lateral opening, and
- both side wings that overlap portions of the outskirt located at both sides of the lateral opening, said side wings extending from both lateral side of the central portion.

The central portion and both side wings of the tab are thus adapted to seal the shield and the outskirt in order to achieve a hermetic inside compartment.

In one particular embodiment, the tab can include two shoulders that respectively link the central portion to each side wing so that while the tab overlaps the lateral opening, both side wings seal the tab over an upper face of the outskirt and the central portion taking place in the lateral opening for closing the outskirt and sealing the shield onto the patient's skin. The shoulders improve the sealing of the tab over the lateral opening and the outskirt. Thereby, all the surface of the central portion can fit the patient's skin.

In still another embodiment, the protecting and connecting device can comprise two receiving holes through the wall of the shield, namely each receiving hole is configured to hold a probe. This provision is suitable for the treatment of the intestinal atresia because the protecting and connecting device can deal with two probes generating an outside derivation of the bowel and then provide a solution for collecting intestinal fluids from the upstream part of the bowel and to progressively enable an infusion into the downstream part of the bowel.

In one embodiment, the first receiving hole can have larger dimensions than the second receiving hole. Given that the cross section of the bowel reduces from the upstream part to the downstream part of the bowel according to the atresia, this feature allows that each probe can be connected to respective sections of the bowel in a suitable manner. Having two receiving holes with different dimensions, thus permits not only to perform a derivation of the bowel but also to keep a flow of the intestinal secretions as natural as possible with each probe fitting the dimensions of the cross section of the bowel where it is connected.

In one embodiment, the protecting and connecting device can comprise a drain probe passing through the first receiving hole of the shield, the drain probe being thus configured to be connected through a first stoma to an upstream part of the patient's intestine.

In one further embodiment, the protecting and connecting device can additionally comprise a feeding probe passing through the second receiving hole of the shield, the feeding probe being thus configured to be connected to a downstream part of the patient's intestine.

According to one embodiment, the protecting and connecting device can comprise a bypass piece that coupling the drain probe with the feeding probe outside of the shield in order to form a derivation of the intestine outside of the patient's body.

In another embodiment, the drain probe and the feeding probe can each comprise a respective connector linked to the bypass piece, each connector including a protective layer. The protective layer allows to preserve the skin integrity of the patient.

According to one other embodiment, the wall of the shield can have an arc shape cross section that defines a top and a base corresponding to the peripheral outline of the shield and said receiving hole can be located on the top of the wall. Said arc shape cross section improves the comfort of the patient notably by avoiding rubbing between the shield and the stoma.

### [BRIEF DESCRIPTION OF THE DRAWINGS]

The present invention is illustrated by way of example, and not by way of limitation, in the figures of the accompanying drawings and in which like reference numerals refer to similar elements and in which:
Fig. 1 is a perspective view of a protecting and connecting device that is compliant with one embodiment of the invention.
Fig. 2 is a top view of the protecting and connecting device illustrated on figure 1.
Fig. 3 is a lateral view of the protecting and connecting device illustrated on figure 1, wherein the tab is getting up for unsticking the protecting and connecting device.
Fig 4. is a perspective view of the protecting and connecting device illustrated on figure 1, wherein the tab is getting up for unsticking the protecting and connecting device.
Fig 5. to Fig. 8 illustrate the steps for setup a derivation of the intestine outside of the body using a protecting and connecting device in compliance with one embodiment of the invention.

### [DESCRIPTION OF PREFERRED EMBODIMENTS]

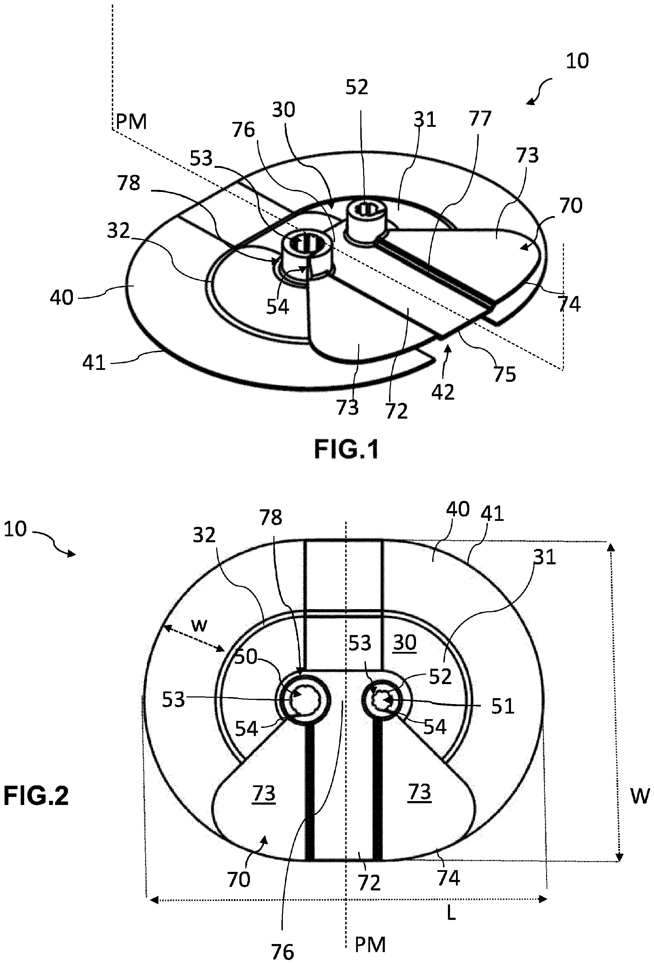

As illustrated by the figures 1 to 8, the invention concerns a protecting and connecting device 10 of at least one stoma site emerging out of the patient skin. The protecting and connecting device is more particularly developed for the treatment of the intestinal atresia, within this application the protecting and connecting device 10 is used for protecting two stomas 20, 21 emerging out of the patient's skin.

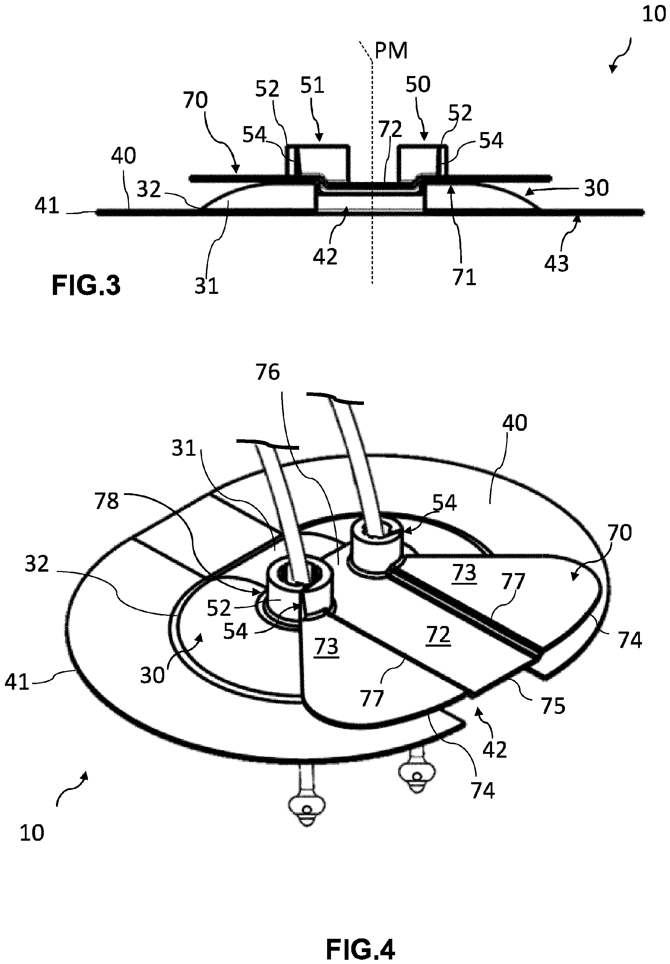

In reference to figures 1 to 4, the device 10 comprises a shield 30 that generates an inside compartment protecting the site of stomas 20, 21. To this end, the shield 30 comprises a wall 31 that is delimited by a peripheral outline 32. As it can be seen on figure 3, the wall 31 of the shield 30 has an arc shape cross section. This shape enables to avoid rubbing between the interne face of the wall 31 and the stomas 20, 21, finally it helps to improve the comfort of the patient 22. The arc shape cross section defines both a top of the shield and a base of the shield 30. Here, the base corresponds to the peripheral outline 32. In the example illustrated by figures 1 to 4, the peripheral outline 32 defines a shield 30 with an oblong shape. Alternatively, the shield 30 can have other kinds of shape like a circular, triangular or rectangular shape.

As illustrated in figures 1 to 4, the device 10 also comprises an outskirt 40. The outskirt 40 surrounds at least partially the peripheral outline 32 of the shield 30. The outskirt 40 extends between the peripheral outline 32 of the shield 30 and peripheral edge 41. The peripheral edge 41 exteriorly delineates the outskirt 40. Indeed, the outskirt 40 can be considered as a strip surrounding the peripheral outline 32 of the shield 30 and be connected to it. In consequence, the outskirt 40 presents the same shape of the peripheral outline 32 which are surrounded by the outskirt 40. Thus, the outskirt 40 includes in one hand two right portions that are opposite to each other. Both right portions define the lateral sides of the device 10. In reference to figure 3, the distance between the peripheral edge 41 of the two right portions defines the width W of the device 10. For example, the width W can be comprised between 30 mm and 100 mm. In the other hand, the outskirt 40 includes two arc shape portions that are opposite to each other. Both arc shape portions define both longitudinal extremities of the device 10. Therefore, the distance between the peripheral edge 41 of said both extremities defines the length L of the device 10. For example, the length of the device 10 can be comprised between 50 mm and 150 mm.

Figures 1, 3 and 4 show that the outskirt 40 comprises a lateral opening 42. More specifically, the lateral opening 42 is located on a right portion of the outskirt 40. The lateral opening 42 is delimited by two lateral edges that extend form the shield 30 to the peripheral edge 41 of the outskirt 40. As it can be seen on figures 1 and 3, the device 10 comprises a transverse median plane PM and the lateral opening 42 which does extend on both sides of said plane PM. The outskirt 40 also includes a lower face 43 which is configured to stick with the patient's skin. To do this, the lower face 43 of the outskirt 40 comprises an adhesive layer for sealing the shield 30 onto the patient's skin. Thus, the device 10 can be considered as a removal patch not only for protecting the emerging site of both stomas 20, 21 but also for keeping a derivation of the bowel that stands outside of the patient's body.

According to one embodiment, the adhesive layer comprises a skin careful adhesive like a medical grade silicone adhesive. However, other adhesives which are classified as medical grade materials can be used. Although it is not illustrated on figures, the adhesive layer is protected by a removal liner. The removal liner is suitable for protecting the adhesive layer during transport and storage of the device 10.

According to embodiments, the lower face 43 of the outskirt 40 defines the contact surface with the patient's skin. In order to improve the patient's comfort, the width w of the strip that forms the outskirt 40 is large. Figure 2 illustrates said strip width w by double arrow. The strip width w can be comprised between 10 and 20 mm. According to another embodiment, the strip width w can be comprised between 13 and 16 mm. Thereby the width w of one strip can represent 20 -40 % of the device 10 width W and 15-30 % of the device 10 length. A large outskirt 40 enables to distribute the linking strength onto a larger surface so that the removal of the device 10 required to provide less energy which allows to reduce irritations of the patient's skin.

The device 10 comprises at least one receiving hole 50, 51 through the wall 31 of the shield 30. In reference to figures 1 to 4, said receiving hole 50, 51 is located on the top of the wall 31. According to one embodiment, said receiving hole 50, 51 defines a passage for at least one probe 60, 61. For the treatment of the intestinal atresia, the device 10 preferably comprises two receiving holes 50, 51. In this embodiment, both receiving holes 50, 51 are arranged both sides of said plane PM. Preferably, the arrangement of both receiving holes 50, 51 is symmetrical relative to said plane PM.

Within this embodiment, a first receiving hole 50 is configured to hold a drain probe 60 while a second receiving hole 51 is configured to hold a feeding probe 61. Figure 4 shows the device 10 on which the probes 60 ,61 are respectively setup through one receiving hole 50, 51.

The device 10 can comprise the drain probe 60 passing through the first receiving hole 50 of the shield 30. According to this arrangement, the drain probe 60 is configured to be connect through a first stoma 20 to an upstream part of the patient's intestine relative to the atresia. The drain probe 60 shunts the intestinal secretions from the intestine toward the derivation 62 which is outside of the body of the patient.

In addition, the device 10 comprises a feeding probe 61 passing through the second receiving hole 51 of the shield 30. The feeding probe 61 is configured to be connected to a downstream part of the patient's intestine relative to the atresia. In this context, the feeding probe 61 reintroduces the intestinal secretions from the derivation 62 into the bowel downstream from the atresia. That permits to shunt the intestinal transit in order to facilitate the healing of bowel after the surgery. The connection between the probes 60, 61 and the bowel is not showed by figures. However, a Pezzer kind probe can be suitable for making the connection with the bowel both upstream and downstream from the atresia.

Figures 1 to 4 show one embodiment in which the first receiving hole 60 has larger dimensions than the second receiving hole 61. The difference in diameter of the first receiving hole 50 and second receiving hole 51 are related to the respective diameter of the secretion tubes passing through the receiving holes 50, 51. Indeed, the cross-section of the drain probe 60 is larger than the cross-section of the feeding probe 61 due to the upstream intestine part that has larger cross-section than the downstream intestine part. For example, the internal diameter of the first receiving hole 50 can be comprised between 12 Fr and 22 Fr, while the internal diameter of the second receiving hole 51 can be comprised between 8 Fr and 16 Fr. According to another embodiment, the first receiving hole 50 and the second receiving hole 51 may have the same dimensions.

As illustrated by figures 1 to 4, each receiving hole 50, 51 comprises a reinforcing structure. In this example, the reinforcing structure includes a cylinder 52. The cylinder 52 surrounds a receiving hole 50, 51 and extends from the peripheral edge of said receiving hole 50, 51 toward the exterior of the shield 30. The cylinder 52 extends according to a determined distance that can be qualified of the height of the cylinder 52. For example, the height of the cylinder 52 can be between 5 mm and 15 mm, more particularly, the height of the cylinder 52 can be 10 mm.

In reference to figures 1 and 2, the cylinder 52 includes ridges 53. Here, the ridges 53 are located on an inside wall of the cylinder 52. The ridges 53 permit to adjust the tube of the probe 60, 61 to the receiving hole 50, 51 and thus it reduces the slipping between the receiving hole 50, 51 and the probe 60, 61.

In addition, each receiving hole 50, 51 can comprise a notch 54 arranged through a peripheral edge of said receiving hole 50, 51. The notch 54 extends the full height of the cylinder 52. More particularly, the notch 54 is arranged by the side of the outskirt 40 where the lateral opening 42 stands. One other particularity, the notch 54 connects with the lateral opening 42. In consequence, the notch 54 is mobile between a closed position and an opened position.

In the closed position, the notch 54 ensures the sealing of the receiving hole 50, 51 with tube of the probe 60, 61. On the contrary, in the opened position the notch 54 enables to engage and to disengage the probes 60, 61 within the receiving holes 50, 51 without compromising the derivation 62 of the intestinal secretions. This feature allows to frequently remove the device 10 for cleaning up both stomas 20, 21, this without removing the derivation.

As shown in figures 1 to 4, the device 10 further comprises a tab 70. The tab 70 does provide a grasp mean for unsealing the shield 30 from the patient's skin. In this embodiment, the tab 70 is connected to one radial portion of the peripheral edge of each said receiving hole 50, 51. Consequently, the tab 70 actuates the notch 54 between its closed position and its opened position. Figures 3 and 4 illustrate that when the tab 70 is lifted, it actuates the notch 54 from its closed position to its opened position. Therefore, the tab 70 permits to unseal the device without using a specific tool.

Figures 1 to 4 illustrate that the tab 70 is configured to overlap and to face at least the lateral opening 42 for ensuring the sealing of the inside compartment of the shield 30. To do this, the tab 70 can include a lower face 71 that is coated by an adhesive layer. The adhesive layer enables to seal the shield 30 to the patient's skin. The adhesive layer can be chosen as described above.

The tab 70 can be considered as a sheet cover of the lateral opening 42. More particularly in one embodiment, the tab 70 includes a central portion 72 that is configured both to overlap and face the lateral opening 42. To do this, in one hand, the central portion 72 is arranged coaxially relative to the lateral opening 42. In other hand, the central portion 72 presents dimensions lightly smaller than the lateral opening 42 in order to take place into it and seal in first place the outskirt 40 and at the same time seal the shield 30.

In addition, the tab 70 has both side wings 73. As illustrated, each side wing 73 extends from one lateral side of the central portion 72. Each side wing 73 is configured to overlap portions of the outskirt 40 that are located at both sides of the lateral opening 42. Overlapping portions of the outskirt 40 on the sides of the lateral opening 42 contribute not only to seal the outskirt 40 to the patient's skin but also to achieve a hermetic inside compartment of the shield 30.

On one hand, said side wings 73 laterally extend from both lateral side of the central portion 72, on the other hand, said wings longitudinally and respectively extend from one receiving hole 50, 51 to their extremal edge portion 74. More specifically, each side wing 73 is linked to one receiving hole 50, 51 through the cylinder 52. The connection on the cylinder 52 radially extends from the notch 54 until the central portion 72. This feature allows to open the notch 54 when the tab 70 is lifted. On figures 1 and to 4, it can be seen the notch 54 begins to open as the tab 70 is lifted. The figures also show that the extremal edge portion 74 presents an arc shape with a curve similar with the arc shape of the outskirt 40. Therefore, the tab 70 perfectly overlaps the outskirt 40 and notably the peripheral edge 41 as it can be seen on figure 2.

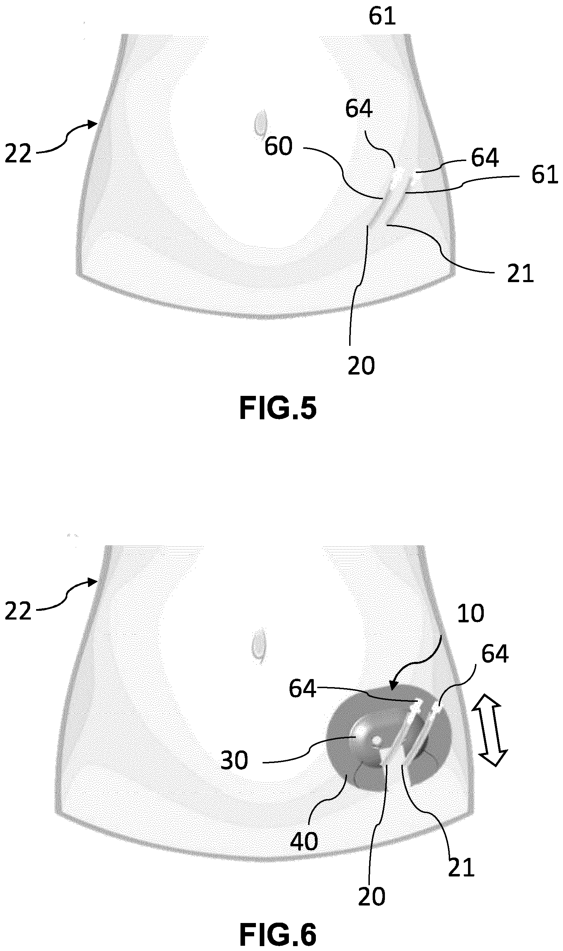

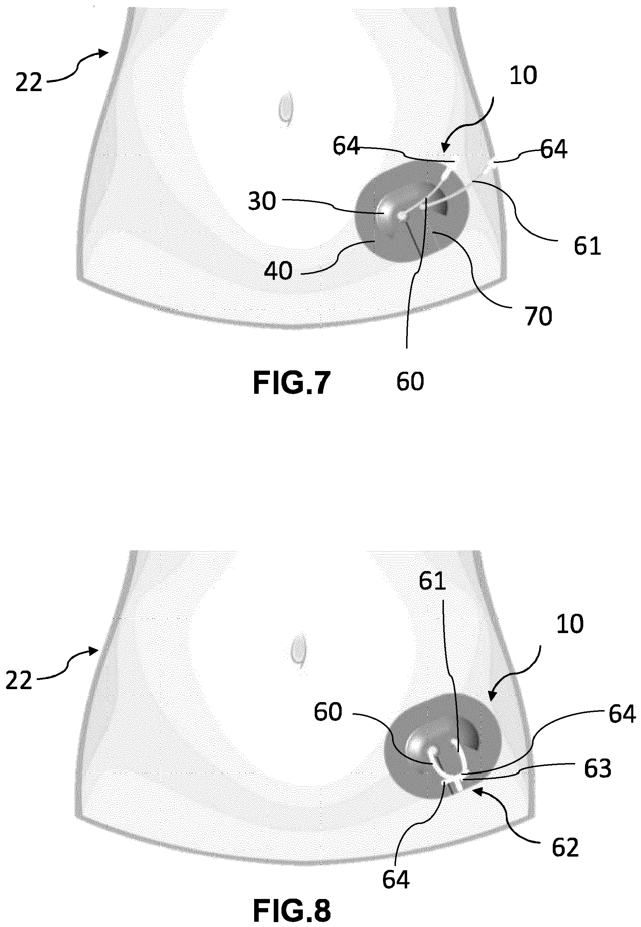

Concerning the central portion 72, it longitudinally extends from one free edge 75 to one support piece 76. The support piece 76 is located on the top of the shield 30 and radially surrounds each receiving hole 50, 51 from the central portion 72 until the notch 54 bypassing the lateral opening 42. Thereby, the tab is like articulated with shield 30 through the support piece 76. Due to this arrangement, as the notch 54, the tab 70 is mobile between a closed position and an opened position. In reference to figure 6, when the tab 70 is in the opened position, the lateral opening 42 and the notch 54 are opened, therefore, it is possible to engage or to disengage the device 10 with the probes 60, 61. On the contrary, figures 7 and 8 show when the tab 70 is in the closed position it seals the shield 30 and the outskirt 40.

As illustrated on figures 1, 2 and 4, the support piece 76 comprises a reinforced band 78 that surrounds the receiving holes 50, 51. The reinforced band 78 is thicker than the other part of the shield 30. The reinforced band 78 rigidifies the shield 30 for respectively keeping and sealing the probes 60, 61 into one receiving hole 50, 51.

In another embodiment, the tab 70 includes two shoulders 77. Each shoulder 77 respectively connects the central portion 72 to each side wing 73 so that while the tab 70 overlaps the lateral opening 42, both side wings 73 sticking with an upper face of the outskirt 40 and the central portion 72 takes place into the lateral opening 42 for closing the outskirt 40 and sealing the shield 30 onto the patient's skin. In closed position, the shoulder 77 fits with a lateral edge that delimits the lateral opening 42. The shoulder 77 permits that all the surface of the central portion 72 fits the patient's skin.

The device 10 can also comprises a bypass piece 63. The bypass piece 63 couples the drain probe 60 with the feeding probe 61 outside of the shield 30. Therefore, through the bypass piece 63 the derivation 62 of the intestine can be established outside of the patient's body. The bypass piece 63 can be constituted by a three ways connector for example in T shape. Namely, two ways can be used for connecting both probes 60, 61 and the third way that can be used for nutrition, sample acquisition, secretion extraction etc. Nevertheless, other kinds of connector can be used for making the bypass piece 63.

Figures 5 to 8 illustrate that both the drain probe 60 and the feeding probe 61 respectively comprise a connector 64. The connector 64 is provided for connecting the bypass piece 63 to the probes 60, 61.

In one embodiment, the device 10 and notably the shield 30, the outskirt 40, the tab 70 can be realized in a flexible material and preferably in a medical grade flexible material. For example, the flexible material can be chosen in the following list of materials: silicone, thermoplastic elastomer e.g., thermoplastic polyurethan (TPU), thermoplastic polyamide (TPA), ethylene vinyl acetate (EVA) or like, or a mixture of those materials.

Figures 5 to 8 illustrate a procedure for setup the device 10 in order to protect the site of two emerging stomas 20 ,21, which can be the case for the treatment of the intestinal atresia.

Focusing on figure 5, wherein both probes 60, 61 are respectively passing through both stomas 20, 21 emerging from the skin of a patient 22. Each probe 60, 61 is connected to the bowel as described above, and a free end of each probe 60, 61 is equipped with a connector 64.

Then figure 6 illustrates how a device 10 according to the invention is placed around both stomas 20, 21. More specifically, while the tab 70 is lifted, the device 10 is placed so that both stomas 20, 21 are positioned in the lateral opening 42 in order to be insert respectively in one hole 50, 51 through one notch 54. A double arrow is represented on figure 6 for illustrating the move to get in or to get out the probes 60, 61 respectively in one hole 50, 51.

In figure 7, both probes 60, 61 are respectively inserted in one hole 50, 51, the tab 70 closing the lateral opening 42 and more generally the device 10 is sealed on the skin of the patient 22. However, the connector 64 of each probe 60, 61 is still free.

Figure 8 illustrates the device 10 fully setup on the skin of the patient 22. Eventually, the connector 64 of both probes 60, 61 is engaged with the bypass piece 63 for creating a shunt of the bowel.

As specified on the foregoing, the unstick 70 allows to remove frequently the device 10 as a patch in order to clean up both stomas 20, 21 without removing the derivation of the bowel. Such an operation can be done once a week for keeping sterile conditions around both stomas 20, 21. Mainly due to hygienic reasons, after each cleaning operation, a new device 10 is placed around both stomas 20, 21. Therefore, the device 10 can be considered as a disposable device.

In one embodiment, the device 10 can be constituted by a single piece. To this end, a plastic injection molding process may be applied. According to this process, an one-shot injection may provide the device 10.

## Claims

1. Protecting and connecting device (10) of at least one stoma site emerging out of a patient's skin, the protecting and connecting device (10) comprising:
- a shield (30) that comprises a wall (31) delimited by a peripheral outline (32), the shield (30) generating an inside compartment protecting the site of said stoma,
- an outskirt (40) surrounding at least partially peripheral outline (32) of the shield (30), wherein a lower face of the outskirt (40) comprises an adhesive layer for sealing the shield (30) onto the patient's skin, and
- at least one receiving hole (50, 51) through the wall (31) of the shield (30), said receiving hole (50, 51) defining a passage for at least one probe (60, 61),
wherein it comprises a tab (70) providing a grasp mean for unsealing the shield (30) from the patient's skin.

2. Protecting and connecting device (10) according to claim 1, wherein said receiving hole (50, 51) comprises a reinforcing structure and a notch (54) arranged through a peripheral edge of said receiving hole (50, 51), the notch (54) being mobile between a closed position and an opened position.

3. Protecting and connecting device (10) according to claim 2, wherein the reinforcing structure includes a cylinder (52) which extends from the peripheral edge of the receiving hole (50, 51) toward the exterior of the shield (30).

4. Protecting and connecting device (10) according to claim 3, wherein the cylinder (52) includes ridges (53) located on an inside wall of the cylinder (52).

5. Protecting and connecting device (10) according to one of claims 2 to 4, wherein the tab (70) is connected to one radial portion of the peripheral edge of said receiving hole (50, 51) so that the tab (70) actuates the notch (54) between the closed position and the opened position.

6. Protecting and connecting device (10) according to one of claims 1 to 5, wherein the outskirt (40) comprising a lateral opening (42) and the tab (70) is configured to overlap and to face at least the lateral opening (42) for establishing the sealing of the inside compartment.

7. Protecting and connecting device (10) according to claims 2 and 6, wherein the lateral opening (42) is in communication with said receiving hole (50, 51) through the notch (54).

8. Protecting and connecting device (10) according to one of claims 1 to 7, wherein the tab (70) including:
- a central portion (72) that overlaps and faces the lateral opening (42), and
- both side wings (73) that overlap portions of the outskirt (40) located at both sides of the lateral opening (42), said side wings (73) extending from both lateral side of the central portion (72).

9. Protecting and connecting device (10) according to claim 8, wherein the tab (70) including two shoulders (77) that respectively link the central portion (72) to each side wing (73) so that while the tab (70) overlaps the lateral opening (42), both side wings (73) seal the tab (70) over an upper face of the outskirt (40) and the central portion (72) taking place in the lateral opening (42) for closing the outskirt (40) and sealing the shield (30) onto the patient's skin.

10. Protecting and connecting device (10) according to one of claims 1 to 9, wherein the tab (70) includes a lower face (71) that is coated by an adhesive layer, which enables to seal the shield (30) to the patient's skin.

11. Protecting and connecting device (10) according to one of claims 1 to 10, wherein it comprises two receiving holes (50, 51) through the wall (31) of the shield (30), each receiving hole (50, 51) is respectively configured to hold a probe (60, 61).

12. Protecting and connecting device (10) according to claim 11, wherein the first receiving hole (50) has larger dimensions than the second receiving hole (51).

13. Protecting and connecting device (10) according to one of claims 11 and 12, wherein it comprises a drain probe (60) passing through the first receiving hole (50) of the shield (30), the drain probe (60) is configured to be connect through a first stoma to an upstream part of the patient's intestine.

14. Protecting and connecting device (10) according to one of claims 11 to 13, wherein it comprises a feeding probe (61) passing through the second receiving hole (51) of the shield (30), the feeding probe (61) is configured to be connect to a downstream part of the patient's intestine.

15. Protecting and connecting device (10) according to claims 13 and 14, wherein it comprises a bypass piece (63) that coupling the drain probe (60) with the feeding probe (61) outside of the shield (30) in order to form a derivation of the intestine outside of the patient's body.

16. Protecting and connecting device (10) according to claim 15, wherein the drain probe (60) and the feeding probe (61) respectively comprise a connector (64) linked to the bypass piece (63).

17. Protecting and connecting device (10) according to one of claims 1 to 16, wherein the wall (31) of the shield (30) has an arc shape cross section that defines a top and a base corresponding to the peripheral outline (32) of the shield (30) and said receiving hole (50, 51) is located on the top of the wall (31).
